# FASCICULE DE BREVET EUROPEEN

(11) **EP 4 416 162 B1**
(45) Date de publication et mention de la délivrance du brevet: **11.03.2026**
(21) Numéro de dépôt: 22802579.7
(22) Date de dépôt: 12.10.2022
(51) Int. Cl.: C07H 1/00, C07H 15/04, C07H 15/10

(54) **PROCÉDÉ DE PRÉPARATION D'ALKYLPOLYGLYCOSIDES FAIBLEMENT COLORÉS AVEC NEUTRALISATION DU MILIEU RÉACTIONNEL APRÈS ÉLIMINATION DU SUCRE**
VERFAHREN ZUR HERSTELLUNG VON NIEDRIG GEFÄRBTEN ALKYLPOLYGLYKOSIDEN MIT NEUTRALISATION DES REAKTIONSMEDIUMS NACH ENTFERNUNG DES ZUCKERS
PROCESS FOR THE PREPARATION OF LOW COLORED ALKYL POLYGLYCOSIDES WITH NEUTRALIZATION OF THE REACTION MEDIUM AFTER REMOVAL OF THE SUGAR

(30) Priorité: 13.10.2021 FR 2110845
(43) Date de publication de la demande: 21.08.2024
(73) Titulaire: Société d'Exploitation de Produits pour les Industries Chimiques SEPPIC, 75321 Paris cedex 07 (FR)
(72) Inventeur: ILLOUS, Estelle, 81100 Castres (FR); DESSILLA, Stéphane, 81100 Castres (FR)
(74) Mandataire: Air Liquide
(86) Numéro de dépôt international: PCT/EP2022/078401
(87) Numéro de publication internationale: WO 2023/062078

(56) Documents cités:
- EP-A1- 0 077 167
- EP-A1- 0 092 876
- EP-A1- 0 338 151
- WO-A1-98/35975
- US-A- 5 576 425
- US-A- 5 681 938

## Description

La présente invention est relative à un procédé de préparation d'Alkyl Polyglycosides peu colorés (couleur inférieur à 1,5 vcs) impliquant des agents de neutralisation de type carbonate.
Les AlkylPolyGlycosides ou APG sont probablement les meilleurs exemples de tensioactifs biosourcés disponibles aujourd'hui sur le marché. Leurs structures moléculaires se caractérisent par la présence simultanée d'une tête hydrophile dérivée de sucres réducteurs (D-glucose, D-xylose ou D-rhamnose sont les sucres réducteurs principalement disponibles à l'échelle industrielle sur le marché) et d'une chaîne hydrocarbonée lipophile plus ou moins longue (cf. formule I : structure simplifiée d'un APG).

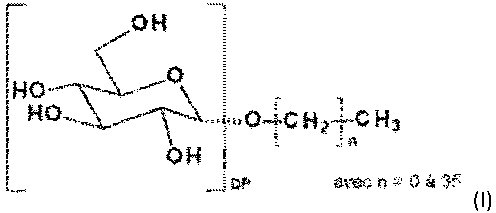

Leur procédé de fabrication à l'échelle industrielle est relativement simple et utilise comme matières premières i) le glucose,le xylose ou le rhamnose cristallisé issus respectivement de l'hydrolyse totale d'amidon de blé, de maïs ou de pomme de terre ou de l'hydrolyse d'hémicelluloses de bois et ii) des alcools gras provenant de la filière oléochimique (hydrogénation d'esters méthyliques issus de la transestérification de triglycérides végétaux). Les réactions de glycosylation de Fischer consistent alors à relier ces deux matières premières entre elles en créant une liaison chimique covalente, comme par exemple dans la réaction (II) entre le glucose et un alcool.

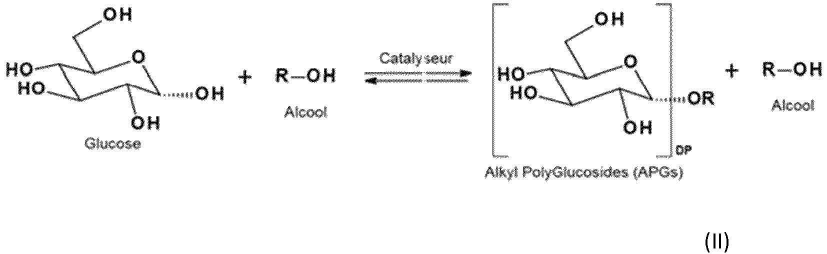

Pour réaliser cette réaction de glycosylation, un catalyseur acide d'origine minérale ou organique est nécessaire et un excès d'alcools est systématiquement introduit jouant ainsi le rôle de réactif et de solvant. En fin de réaction, les APG se retrouvent dispersés ou solubilisés dans l'excès d'alcool qui n'a pas réagi.

Les APG se distinguent par la nature de la chaîne alkyle hydrocarbonée R ainsi que par leur Degré de Polymérisation moyen DP supérieur à 1 mais inférieur ou égal à 2,5.

A l'issue de la phase réactionnelle de glycolysation, une étape de neutralisation est opérée afin de désactiver le catalyseur et de stopper la réaction.

Selon la longueur de la chaîne alkyle de l'alcool et l'utilisation associée, ledit alcool est soit éliminé, soit conservé.

L'étape de neutralisation diffère selon la longueur de la chaîne alkyle hydrocarbonée. Dans le cas où celle-ci présente un nombre d'atomes de carbone inférieur à 12, la neutralisation est réalisée par une solution aqueuse d'hydroxyde de sodium. L'excès d'alcools gras présent en fin de glycosylation est ensuite éliminé par distillation sous vide poussé ou distillation moléculaire, ou par évaporation, généralement, à l'aide d'un évaporateur couche mince à film tombant, ou d'un évaporateur couche mince à court trajet, et le concentré d'APG recueilli est finalement solubilisé dans l'eau. Les produits commerciaux ainsi obtenus se présentent donc sous la forme de solutions aqueuses d'APG avec une concentration massique comprise entre 40 et 80%.

Dans le cas où la chaîne alkyle hydrocarbonée R présente un nombre d'atome de carbone supérieur ou égal à 12 , la neutralisation est généralement réalisée par l'hydroxyde de sodium ou par l'hydroxyde de potassium, seuls ou en combinaison avec un agent réducteur tel que dans le Brevet Européen publié sous le numéro EP0077167, dans la demande de Brevet Européen publiée sous le numéro EP0338151 A1, dans le Brevet Européen EP0388857 B1, comme par exemple le borohydrure de sodium (NaBH₄) ou l'hypophosphite de sodium (NaH₂PO₂). Le mélange d'APG et de l'excès d'alcools gras est isolé après neutralisation et est commercialisé tel quel. La proportion massique APG et des Alcools gras dépend directement de la stœchiométrie molaire retenue au départ pour les matières premières et de leur réactivité. Toutefois, des proportions de 5 à 30% massique d'APG et de 70 à 95% d'alcools gras sont généralement observées. Les produits correspondants sont donc solides sous forme d'écailles ou de perles, ou sous une forme liquide, en fonction de la nature de la chaîne alkyle hydrocarbonée R.

Cependant, l'étape de neutralisation des APG émulsionnants dont la chaîne alkyle hydrocarbonée R comporte un nombre d'atomes de carbone supérieur ou égal à 12 par une base de l'état de la technique (exemple NaOH, KOH), pour atteindre une valeur du pH d'une dispersion à 5% massique dans l'eau du milieu neutralisé comprise entre 5,5 et 7,5, engendre une coloration importante du produit.

Par "mesure du pH d'une dispersion à 5% massique dans l'eau", on désigne au sens de la présente invention la méthode analytique de mesure de pH d'une dispersion d'une composition à base d'APG selon les dispositions de la norme NF EN 1262, ladite mesure est réalisée par mesure potentiométrique à l'aide d'une électrode pH (milieux aqueux) combinée et d'un pH mètre.

Cette coloration peut altérer les qualités organoleptiques des produits finis dans lesquels les compositions contenant les APG sont introduites. C'est pour cela que des solutions sont apportées pour minimiser la coloration des compositions contenant les APG dont la chaîne hydrocarbonée R comporte un nombre d'atomes de carbone supérieur ou égal à 12. Deux leviers connus de l'état de la technique sont classiquement employés pour obtenir de telles compositions à base d'APG dont la chaîne alkyle hydrocarbonée R comporte un nombre d'atomes de carbone supérieur ou égale à 12 peu colorées (< 1,5 vcs).

Par "composition peu colorée", on désigne au sens de la présente invention une composition dont l'indice de couleur Gardner, tel que défini par la norme DIN-ISO 4630, est inférieur ou égal à 1,5 VCS. L'indice de couleur Gardner est mesuré en utilisant un colorimètre LICO 200/Dr LANGE (ou équivalent) qui effectue des mesures par transmission de la lumière sur tout milieu. Un tel colorimètre fonctionne avec une lampe halogène correspondant à l'illuminant normalisé C, défini par la norme DIN 5033 et avec un observateur normalisé 2°. Pendant la mesure, un faisceau de rayonnement de référence compense les variations des valeurs enregistrées dues aux différences de lampe et de température.

Le premier levier consiste à associer un agent réducteur à la base utilisée. Parmi ces agents réducteurs, nous pouvons citer le borohydrure de sodium (NaBH₄) ou de l'hypophosphite de sodium (NaH₂PO₂). Cette solution n'est pas entièrement satisfaisante En effet, bien que très efficace pour minimiser la coloration de la composition traitée, le NaBH₄ est un agent réducteur dangereux à manipuler et à mettre en œuvre (produit corrosif, dégagement d'hydrogène ). Le NaH₂PO₂ est quant à lui très peu efficace même s'il est introduit à forte concentration.

Le second levier couramment utilisé et décrit dans l'état de la technique, pour minimiser la couleur des compositions à base d'APG, dont la chaîne alkyle hydrocarbonée R comporte un nombre d'atome de carbone supérieur ou égal à 12, est la réalisation d'une décoloration au péroxyde d'hydrogène (H₂O₂) lors de l'étape de finition. Bien qu'efficace, cette étape est néanmoins fastidieuse car elle nécessite d'ajuster la valeur du pH d'une dispersion à 5% massique dans l'eau entre 7,0 et 7,5 tout en maintenant le pouvoir oxydant du milieu par ajouts de d'H₂O₂. Cette étape délicate à réaliser peut durer plusieurs heures et donc accroître significativement la durée de production, en diminuant la productivité.

D'autres documents divulguent d'autres procédés de préparation. Ainsi, EP0092876A1 décrit une préparation par transglycosylation comprenant une étape de neutralisation réalisée avec Na2CO3. US5681938A décrit un procédé de préparation d'APG comprenant une étape de réalisation réalisée avec une amine tertiaire. US5576425A divulgue une étape de neutralisation incluant des carbonates avec MgO comme base préférée. Enfin WO98/35975A1 décrit une préparation d'APG comprenant l'utilisation d'un catalyseur sulfate binaire lui-même étant un mélange de H2SO4 et d'une base inorganique incluant les carbonates.Le problème technique à résoudre est donc de trouver une alternative à la neutralisation des compositions à base d'APG dont la chaîne alkyle hydrocarbonée R comporte un nombre d'atomes de carbone supérieur ou égale à 12. Cette alternative doit être efficace et facile à mettre en œuvre, tout en garantissant une couleur inférieure ou égale à 1,5 vcs sans étape de décoloration.

Une solution de la présente invention est un procédé de préparation d'une composition (C) de couleur inférieure ou égale à 1,5 vcs, comprenant pour 100% de sa masse :
(i) une quantité supérieure ou égale à 40% massique et inférieure ou égale à 95% massique, de préférence supérieure ou égale à 50% massique et inférieure ou égale à 95% massique, encore plus préférentiellement supérieure ou égale à 70% massique et inférieure ou égale à 90% massique d'un alcool de formule (I) :
   R-OH (I), dans laquelle R représente un radical hydrocarboné, linéaire ou ramifié, saturé ou insaturé, pouvant comporter au moins un fonction hydroxy, et comportant de douze à vingt-deux atomes de carbone, ou d'un mélange d'alcools de formule (I) ;
(ii) une quantité supérieure ou égale à 5% massique et inférieure ou égale à 60% massique, de préférence supérieure ou égale à 5% massique et inférieure ou égale à 50% massique, et encore plus préférentiellement supérieure ou égale à 10% et inférieure ou égale à 30% massique d'une composition (C1) représentée par la formule (II) :
   R-O-(G)x-H (II), dans laquelle le reste G représente le reste d'un sucre réducteur, R représente un radical tel que défini dans la formule (I) et x, qui indique le degré moyen de polymérisation du reste G représente un nombre décimal supérieur à 1,05 et inférieur ou égal à 2,5, ou d'un mélange de compositions (C1) de formule (II); étant entendu que la somme des proportions massiques des composés et des compositions (I) et (II) est égale à 100% massique,
ledit procédé comprenant successivement :
a) Une étape a) de glycosylation, consistant en une réaction entre au moins un alcool de formule (I) et au moins un sucre réducteur de formule (III), : H-O-(G)-H (III), en présence d'au moins un catalyseur acide (CA), à une température supérieure ou égale à 100°C et inférieure ou égale à 120°C, de préférence supérieure ou égale à 100°C et inférieure ou égale à 115°C, encore plus préférentiellement supérieure ou égale à 100°C et inférieure ou égale à 110°C,
   le au moins un catalyseur acide (CA) étant choisi parmi les éléments du groupe constitué par l'acide sulfurique, l'acide chlorhydrique, l'acide phosphorique, l'acide nitrique, l'acide hypophosphoreux, l'acide méthane-sulfonique, l'acide para-toluène sulfonique, l'acide trifluorométhane sulfonique et les résines échangeuses d'ions acides,
b) Une étape b) d'élimination du sucre réducteur de formule (III), qui n'a pas réagi à l'étape a), du milieu réactionnel,
c) Une étape c) de neutralisation du milieu réactionnel issu de l'étape b) avec une solution aqueuse comprenant un agent basique (Ab) choisi parmi les éléments du groupe constitué par :
   ▪ les carbonates de formule (IVa) :

      XnCO₃ (IVa),

      dans laquelle X représente un atome de sodium ou de potassium et n est un nombre entier égal à 2, ou X représente un atome de calcium ou un atome de magnésium et n est un nombre entier égal à 1, ou
   ▪ les hydrogénocarbonates de formule (IVb) :

      Y(HCO₃)m (IVb),

      dans laquelle Y représente un atome de sodium ou de potassium et m est un nombre entier égal à 1, ou Y représente un atome de calcium ou un atome de magnésium et m est un nombre entier égal à 2,
   la neutralisation étant réalisée de manière à obtenir un milieu réactionnel dont la dispersion à 5% massique dudit milieu réactionnel dans l 'eau présente une valeur de pH comprise entre 5,5 et 7,5, et
d) Une étape d) de récupération d'au moins une composition (C) de couleur inférieure ou égale à 1,5 vcs.

L'indice de couleur caractérisant la composition (C) préparée selon le procédé objet de la présente invention, est l'indice de couleur Gardner, tel que défini par la norme DIN-ISO 463. L'indice de couleur Gardner est mesuré en utilisant un colorimètre LICO 200/Dr LANGE (ou équivalent) qui effectue des mesures par transmission de la lumière sur tout milieu. Un tel colorimètre fonctionne avec une lampe halogène correspondant à l'illuminant normalisé C, défini par la norme DIN 5033 et avec un observateur normalisé 2°. Pendant la mesure, un faisceau de rayonnement de référence compense les variations des valeurs enregistrées dues aux différences de lampe et de température.

L'unité d'expression de l'indice de couleur Gardner, caractérisant la composition (C) préparée selon le procédé objet de la présente invention, est le VCS.

Selon le cas, le procédé selon l'invention peut présenter une ou plusieurs des caractéristiques suivantes :
- ladite composition (C1) consistant en un mélange de composés représentés par les formules (II1), (II2), (II3), (II4) et (II5):

   R-O-(G)1-H (II1),

   R-O-(G)2-H (II2),

   R-O-(G)3-H (II3),

   R-O-(G)4-H (II4),

   R-O-(G)5-H (II5),

   dans les proportions molaires respectives a1, a2, a3, a4 et a5, telles que:
   ▪ la somme: a1+ a2 + a3 + a4 + a5 est égale à 1, et
   ▪ la somme a1 + 2a2 + 3a3 + 4a4 + 5a5 est égale à x ;
- la composition (C) comprend une quantité inférieure ou égale à 2% massique, de préférence inférieure ou égale à 1% massique du sucre réducteur de formule (III) :

   H-O-(G)-H (III) ;

   étant entendu que la somme des proportions massiques des composés et des compositions (I), (II) et (III) est égale à 100% massique ;
- l'agent basique (Ab) présent dans la solution aqueuse est le carbonate de sodium de formule (IVa) dans laquelle X représente l'atome de sodium et n est égal à 2 ;
- à l'étape c) la solution aqueuse de l'agent basique (Ab) comprend entre 10 % et 25% massique dudit agent basique (Ab) ;
- l'étape c) est réalisée en deux temps : un premier temps durant lequel la neutralisation est réalisée de manière à obtenir un milieu réactionnel dont une dispersion à 5% massique dans l'eau présente une valeur du pH comprise entre 3,5 et 5,5 avec une solution d'hydroxyde de sodium (NaOH) ou d'hydroxyde de potassium (KOH), puis un deuxième temps durant lequel la neutralisation est réalisée de manière à obtenir un milieu réactionnel dont une dispersion à 5% massique dans l' eau présente une valeur du pH compris entre 5,5 et 7,5 avec une solution aqueuse d'agent basique (Ab) ;
- le sucre réducteur de formule (III) choisi pour la glycolysation de l'étape a) est choisi parmi les éléments du groupe constitué par le glucose, le xylose, l'arabinose et le rhamnose ;
- l'étape b) d'élimination du sucre réducteur de formule (III) est réalisée par filtration, centrifugation ou décantation ;
- l'étape a) comprend les sous-étapes successives suivantes :
   i) Introduction d'un alcool de formule (I) dans un réacteur (Ré) équipé d'une agitation mécanique et d'un dispositif de mise sous vide ;
   ii) Chauffage de l'alcool de formule (I) à une température comprise entre 80°C et 90°C. sous agitation mécanique ;
   iii) Chargement du sucre réducteur de formule (III) dans le réacteur (Ré) ;
   iv) Introduction d'au moins un catalyseur (CA) dans le réacteur (Ré),
   v) Chauffage sous vide du milieu réactionnel présent dans le réacteur (Ré) à une température comprise entre 100°C et 110°C pendant la durée de la réaction, et
   vi) Refroidissement du milieu issu de la sous-étape v) à une température comprise entre 70°C et 80°C ;
- dans la formule (I) et/ou dans la formule (II) le radical R est choisi parmi les radicaux suivants : lauryle (ou n-dodécyle) myristyle (ou n-tétradécyle ), n-pentadécyle, cétyle (ou n-hexadécyle), n-heptadécyle, stéaryle (ou n-octadécyle), palmitoléyle (ou 9-hexadécényle), oléyle (ou 9-octadécényle), linoléyle (9,12-octadecadiényle), linolényle (ou 6,9,12-octadécatriényle) nonadécyle, arachidyle (ou n-eicosyle), béhényle (ou n-docosyle), érucyle (13-docosényle), ou 12-hydroxystéaryle ;
- le radical R est choisi parmi les radicaux suivants : 2-hexyl octyle, 2-hexyl décyle, 2-hexyl dodécyle, 2-octyl décyle, 2-octyl dodécyle, 2-décyl tétradécyle, isostéaryle (ou 16-méthyl heptadécyle) ou isomyristyle (ou 13-méthyl tridécyle) ;
- pendant les sous-étapes ii) à iv) le réacteur (Ré) est inerté sous azote ;
- le procédé comprend entre les sous-étapes ii) et iii) une étape de mise sous vide, de préférence à une pression inférieure ou égale à 50 millibars.
- la sous-étape vi) est réalisée à pression atmosphérique.

L'emploi de carbonates de formule (IVa) ou d'hydrogénocarbonates de formule (IVb) ne contribue pas à augmenter la couleur de la composition (C) (la présence d'un agent réducteur n'étant pas alors nécessaire) tout en neutralisant au pH désiré d'une dispersion à 5% massique de la composition (C) (dont la valeur est comprise entre 5,5 et 7,5). L'utilisation d'un agent basique (Ab) permet d'éviter une étape de décoloration impliquant l'utilisation d'un agent péroxyde, ou autres, puisqu'elle permet d'atteindre une couleur inférieure ou égale à 1,5 vcs. Par sucre réducteur, on désigne dans la définition de la formule (II) et dans la définition de la formule (III), les dérivés saccharidiques qui ne présentent pas dans leurs structures de liaison glycosidique établie entre un carbone anomérique et l'oxygène d'un groupement acétal tels qu'ils sont définis dans l'ouvrage de référence : « Biochemistry », Daniel Voet/Judith G. Voet, p. 250, John Wyley & Sons, 1990.

La structure oligomérique (G)x présente dans la formule (II), peut se présenter sous toutes formes d'isoméries, qu'il s'agisse d'isomérie optique, d'isomérie géométrique ou d'isomérie de position ; elle peut aussi représenter un mélange d'isomères.

Dans la formule (II) telle que définie ci-dessus, le radical R est lié à G par le carbone anomérique du reste saccharide, de manière à former une fonction acétal.

Selon un aspect particulier de la présente invention, dans la définition des composés de formules (II) et de formule (III), G représente le reste d'un sucre réducteur choisi parmi le glucose, le dextrose, le saccharose, le fructose, l'idose, le gulose, le galactose, le maltose, l'isomaltose, le maltotriose, le lactose, le cellobiose, le mannose, le ribose, le xylose, l'arabinose, le lyxose, l'allose, l'altrose, le rhamnose, dextrane ou le tallose.

Selon un aspect particulier de la présente invention, dans la définition des composés de formule (II), G représente le reste d'un sucre réducteur choisi parmi les restes du glucose, du xylose, de l'arabinose ou du rhamnose, et x représente un nombre décimal supérieur ou égal à 1,05 et inférieur ou égal à 2,5.

Selon un aspect encore plus particulier de la présente invention, dans la définition des composés de formule (II), G représente le reste d'un sucre réducteur est choisi parmi les restes du glucose, du xylose, de l'arabinose ou du rhamnose, et x représente un nombre décimal supérieur ou égal à 1,05 et inférieur ou égal à 2,0, et encore plus particulièrement supérieur ou égal à 1,25 et inférieur ou égal à 2,0.

Selon un autre aspect particulier de la présente invention, le sucre réducteur de formule (III) est choisi parmi les éléments du groupe constitué par le glucose, le dextrose, le saccharose, le fructose, l'idose, le gulose, le galactose, le maltose, l'isomaltose, le maltotriose, le lactose, le cellobiose, le mannose, le ribose, le xylose, l'arabinose, le lyxose, l'allose, l'altrose, le rhamnose, dextrane ou le tallose.

Selon un aspect plus particulier de la présente invention, le sucre réducteur de formule (III) est choisi parmi le glucose, le xylose, l'arabinose ou le rhamnose.

Le procédé selon l'invention consiste à réaliser l'étape de filtration/centrifugation avant de faire la neutralisation du produit par une solution aqueuse comprenant un agent basique (Ab).

Selon un aspect particulier, le procédé a pour objet la préparation d'une composition (C) de couleur inférieure ou égale à 1,5 VCS, comprenant pour 100% de sa masse :
- de 45% à 55% massique d'un mélange (M1) d'alcools de formule (I) comprenant pour 100% de la masse dudit mélange (M1), 50% massique d'un alcool de formule (I) dans laquelle R représente le radical n-hexadécyle et 50% massique d'un alcool de formule (I) dans laquelle R représente le radical n-octadécyle,
- de 45% à 54% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical n-hexadécyle et le radical n-octadécyle,
- moins de 1% massique de glucose.

Selon un aspect particulier, le procédé a pour objet la préparation d'une composition (C) de couleur inférieure ou égale à 1,5 VCS, comprenant pour 100% de sa masse :
- de 45% à 55% massique d'un mélange (M'1) d'alcools de formule (I) comprenant pour 100% de la masse dudit mélange (M'1), 70% massique d'un alcool de formule (I) dans laquelle R représente le radical n-hexadécyle et 30% massique d'un alcool de formule (I) dans laquelle R représente le radical n-octadécyle
- de 45% à 54% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical n-hexadécyle et le radical n-octadécyle
- moins de 1% massique de glucose.

Selon un aspect particulier, le procédé a pour objet la préparation d'une composition (C) de couleur inférieure ou égale à 1,5 VCS, comprenant pour 100% de sa masse :
- de 75% à 90% massique d'un mélange (M"1) d'alcools de formule (I) comprenant pour 100% de la masse dudit mélange (M"1), 50% massique d'un alcool de formule (I) dans laquelle R représente le radical n-hexadécyle et 50% massique d'un alcool de formule (I) dans laquelle R représente le radical n-octadécyle
- de 10% à 24% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical n-hexadécyle et le radical n-octadécyle
- moins de 1% massique de glucose.

Selon un aspect particulier, le procédé a pour objet la préparation d'une composition (C) de couleur inférieure ou égale à 1,5 VCS, comprenant pour 100% de sa masse :
- de 75% à 90% massique d'un mélange (M‴1) d'alcools de formule (I) comprenant pour 100% de la masse dudit mélange (M‴1), 70% massique d'un alcool de formule (I) dans laquelle R représente le radical n-hexadécyle et 30% massique d'un alcool de formule (I) dans laquelle R représente le radical n-octadécyle
- de 10% à 24% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical n-hexadécyle et le radical n-octadécyle
- moins de 1% massique de glucose.

Selon un aspect particulier, le procédé a pour objet la préparation d'une composition (C) de couleur inférieure ou égale à 1,5 VCS, comprenant pour 100% de sa masse :
- de 75% à 90% massique d'un alcool de formule (I) dans laquelle R représente le radical n-tétradécyle
- de 10% à 24% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical n-tétradécyle
- moins de 1% massique de glucose.

Selon un aspect particulier, le procédé a pour objet la préparation d'une composition (C) de couleur inférieure ou égale à 1,5 VCS, comprenant pour 100% de sa masse :
- de 75% à 90% massique d'un mélange d'alcools de formule (I) dans laquelle R représente le radical n-dodécyle, le radical n-tétradécyle, le radical n-hexadécyle et le radical n-octadécyle
- de 10% à 24% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical représente le radical n-dodécyle, le radical n-tétradécyle, le radical n-hexadécyle, et le radical n-octadécyle
- moins de 1% massique de glucose.

Selon un aspect particulier, le procédé a pour objet la préparation d'une composition (C) de couleur inférieure ou égale à 1,5 VCS, comprenant pour 100% de sa masse :
- de 75% à 90% massique d'un mélange d'alcools de formule (I) dans laquelle R représente le radical n-eicosyl et le radical n-docosyle
- de 10% à 24% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical n-eicosyl et le radical n-docosyle
- moins de 1% massique de glucose.

Selon un aspect particulier, le procédé a pour objet la préparation d'une composition (C) de couleur inférieure ou égale à 1,5 VCS, comprenant pour 100% de sa masse :
- de 75% à 90% massique d'un mélange d'alcools de formule (I) dans laquelle R représente le radical n-dodécyle, le radical n-tétradécyle, le radical n-hexadécyle, le radical n-eicosyl et le radical n-docosyle
- de 10% à 24% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical n-dodécyle, le radical n-tétradécyle, le radical n-hexadécyle, le radical n-eicosyl et le radical n-docosyle
- moins de 1% massique de glucose.

Selon un aspect particulier, le procédé a pour objet la préparation d'une composition (C) de couleur inférieure ou égale à 1,5 VCS, comprenant pour 100% de sa masse :
- de 70% à 90% massique d'un mélange d'alcools de formule (I) dans laquelle R représente le radical n-dodécyle, le radical n-tétradécyle, le radical n-hexadécyle, le radical n-eicosyl et le radical n-docosyle
- de 10% à 29% massique d'au moins une composition (C1) représentée par la formule (II) dans laquelle G représente le radical xylosyl ou α,β-D-xylopyranosyl, obtenu à partir de la suppression du groupe hydroxyl hémiacétal du α,β-D-xylopyranose, x représente un nombre décimal supérieur ou égal à 1,05 et inférieur ou égal à 2,0, R représente le radical 2-octyl dodécyle
- moins de 1% massique de xylose.

Selon un aspect particulier, l'agent basique (Ab) présent dans la solution aqueuse est le carbonate de potassium de formule (IVa) dans laquelle X représente l'atome de potassium et n est égal à 2.

Selon un aspect particulier, l'agent basique (Ab) présent dans la solution aqueuse est l'hydrogénocarbonate de sodium de formule (IVb) dans laquelle Y représente un atome de sodium et m est égal à 1.

Selon un aspect particulier, le catalyseur acide (CA) est choisi parmi les éléments du groupe constitué par l'acide sulfurique, l'acide phosphorique, l'acide hypophosphoreux, l'acide méthane-sulfonique, l'acide p-toluène sulfonique.

### EXEMPLES

### A. Comparaison de l'effet de l'agent basique (Ab) sur la couleur d'une composition d'alcools gras et d'alkylpolyglucosides lorsque l'agent basique (Ab) neutralisant utilisé est un carbonate selon l'invention ou la soude ( agent neutralisant comparatif).

Des comparaisons entre un carbonate et la soude ont été effectuées en fonction de la gamme de pH suivant la sous-étape de filtration du milieu réactionnel.

Le tableau 1 suivant rassemble des résultats de neutralisation mettant en jeu le milieu réactionnel filtré obtenu à partir de réactions de glycosylations entre du glucose cristallisé et de différents alcools gras sous forme de coupes ou purs : coupe cétéarylique C-16/18, tétradécanol-1 (alcool C14), dodécanol-1 (alcool C12) . Les différents paramètres étudiés sont la nature de l'agent de pré-neutralisation (valeur entre 3,5 et 5,5 du pH de la dispersion à 5% massique dans l'eau du milieu post-filtration et de neutralisation, valeur entre 5,5 et 7,5 du pH de la dispersion à 5% massique dans l'eau du milieu (Na₂CO₃ et/ou NaOH).

### 1. Exemples selon l'invention

### Exemple 1.1 Coupe d'alcools 16/18 et Na₂CO₃ comme agent neutralisant selon l'invention

### ETAPE 1 : Réaction de Glycosylation :

529,3 g d'alcool cétéarylique (C-16/18) sont chargés dans un réacteur équipé d'une agitation mécanique et d'un montage de distillation sous vide. L'alcool est fondu à 85°C et mis sous agitation et sous barbotage d'azote. Le milieu est mis sous vide à des pressions inférieures à 50 millibars. 69,5 g de glucose anhydre sous forme de poudre sont ajoutés. Le milieu est inerté sous azote. Pour démarrer la réaction d'éthérification (glycosylation) 0,5 g d'une solution aqueuse d'acide hypophosphoreux (H₃PO₂) à 50 % puis 0,6 g d'une solution aqueuse d'acide sulfurique (H₂SO₄) à 98 % sont ajoutés et la température est augmentée et maintenue à 105°C. La réaction est poursuivie pendant 5h45.

### ETAPE 2 : Filtration et Neutralisation du milieu réactionnel :

Le milieu est refroidi à 80°C à pression atmosphérique puis filtré sur filtre cloche équipé d'une plaque (3-6 µm) afin d'éliminer le sucre résiduel. 167 g de produit ainsi obtenu est ensuite réintroduit en réacteur à 85°C puis neutralisé en ajoutant sous agitation 1,7 g d'une solution aqueuse de Na₂CO₃ à 10 % pour atteindre une composition référencée (Composition 1)

### Analyses :

- La valeur du pH d'une dispersion à 5% massique dans l'eau de la (Composition 1) est de 6,4, et
- la mesure de la couleur de (Composition 1) est de 0,9 VCS.

### Exemple 1.2 Coupe d'alcools 16/18 et NaOH comme agent pré-neutralisant et Na₂CO₃ comme agent neutralisant selon l'invention

### ETAPE 1 : Réaction de Glycosylation :

529,3 g d'alcool cétéarylique (C-16/18) sont chargés dans un réacteur équipé d'une agitation mécanique et d'un montage de distillation sous vide. L'alcool est fondu à 85°C et mis sous agitation et sous barbotage d'azote. Le milieu est mis sous vide à des pressions inférieures à 50 millibars. 69,5 g de glucose anhydre sous forme de poudre sont ajoutés. Le milieu est inerté sous azote. Pour démarrer la réaction d'éthérification (glycosylation) 0,5 g d'une solution aqueuse de H₃PO₂ à 50 % puis 0,6 g d'une solution aqueuse de H₂SO₄ à 98 % sont ajoutés et la température est augmentée et maintenue à 105°C. La réaction est poursuivie pendant 5h45.

### ETAPE 2 : Filtration et Neutralisation du milieu réactionnel :

Le milieu est refroidi à 80°C à pression atmosphérique puis filtré sur filtre poche (100 µm) afin d'éliminer le sucre résiduel. 264,4 g de produit ainsi obtenu est ensuite réintroduit en réacteur à 75°C puis pré-neutralisé en introduisant sous agitation 0,56 g d'une solution aqueuse de NaOH à 25 % pour atteindre une valeur de pH égale à 4,1 pour une dispersion à 5% massique dans l'eau du milieu présent dans le réacteur et une couleur de 1,6 VCS.

Le produit est ensuite neutralisé toujours sous agitation à 75°C en introduisant 0,9 g d'une solution aqueuse de Na₂CO₃ à 10 % pour obtenir une composition référencée (Composition 2).

### Analyses :

- La valeur du pH d'une dispersion à 5% massique dans l'eau de la (Composition 2) est de 6,4, et
- la mesure de la couleur de (Composition 2) est de 1,0 VCS.

### Exemple 1.3 tétradécanol-1 (alcool myristylique) et NaOH comme agent pré-neutralisant et Na₂CO₃ comme agent neutralisant selon l'invention

### ETAPE 1 : Réaction de Glycosylation :

355,8 g d'alcool myristylique (tétradécanol-1) sont chargés dans un réacteur équipé d'une agitation mécanique et d'un montage de distillation sous vide. L'alcool myristique est fondu à 85°C et mis sous agitation et sous barbotage d'azote. Le milieu est mis sous vide à 25 millibars. 49,7 g de glucose anhydre sous forme de poudre sont ajoutés. Le milieu est inerté sous azote. Pour démarrer la réaction d'éthérification (glycosylation) 0,38 g d'une solution aqueuse de H₃PO₂ à 50 % puis 0,61 g d'une solution aqueuse de H₂SO₄ à 98 % sont ajoutés et la température est augmentée et maintenue à 105°C. La réaction est poursuivie pendant 5h00.

### ETAPE 2 : Neutralisation du milieu réactionnel :

Le milieu est refroidi à 80°C à pression atmosphérique puis filtré sur filtre K1 (4 µm) afin d'éliminer le sucre résiduel. 164 g de produit est ensuite réintroduit en réacteur à 80°C puis pré-neutralisé en introduisant 0,46 g d'une solution aqueuse de NaOH à 25 % sous agitation pour atteindre une valeur de pH de 6,0 pour une dispersion à 5% massique dans l'eau du milieu présent dans le réacteur.

Le produit est finalement neutralisé à 80°C en réacteur sous agitation, en introduisant 0,29 g d'une solution aqueuse de Na₂CO₃ à 10 % pour obtenir une composition référencée (Composition 3).

### Analyses :

- La valeur du pH d'une dispersion à 5% massique dans l'eau de la (Composition 3) est de 6,6, et
- la mesure de la couleur de (Composition 3) est de 1,1 VCS.

### Exemple 1.4 tétradécanol-1 (alcool myristylique) et Na₂CO₃ comme agent neutralisant selon l'invention

### ETAPE 1 : Réaction de Glycosylation :

355,8 g d'alcool myristylique (tétradécanol-1) sont chargés dans un réacteur équipé d'une agitation mécanique et d'un montage de distillation sous vide. L'alcool est fondu à 85°C et mis sous agitation et sous barbotage d'azote. Le milieu est mis sous vide à 25 millibars. 49,7 g de glucose anhydre sous forme de poudre sont ajoutés. Le milieu est inerté sous azote. Pour démarrer la réaction d'éthérification (glycosylation) 0,38 g d'une solution aqueuse de H₃PO₂ à 50 % puis 0,61 g d'une solution aqueuse de H₂SO₄ à 98 % sont ajoutés et la température est augmentée et maintenue à 105°C. La réaction est poursuivie pendant 5h00.

### ETAPE 2 : Neutralisation du milieu réactionnel :

Le milieu est refroidi à 80°C à pression atmosphérique puis filtré sur filtre K1 (4 µm) afin d'éliminer le sucre résiduel. 160,5 g de produit est ensuite réintroduit en réacteur à 70°C puis neutralisé en ajoutant sous agitation 1,77 g d'une solution aqueuse de Na₂CO₃ à 10 % pour atteindre une composition référencée (Composition 4).

### Analyses :

- La valeur du pH d'une dispersion à 5% massique dans l'eau de la (Composition 4) est de 7,4, et
- la mesure de la couleur de (Composition 4) est de 1,0 VCS.

### Exemple 1.5 dodécanol-1 (alcool laurique) et Na₂CO₃ comme agent neutralisant selon l'invention

### ETAPE 1 : Réaction de Glycosylation :

190,6 g d'alcool laurylique (dodécanol-1) sont chargés dans un réacteur équipé d'une agitation mécanique et d'un montage de distillation sous vide. L'alcool est fondu à 85°C et mis sous agitation et sous barbotage d'azote. Le milieu est mis sous vide à 30 millibars. 26,6 g de glucose anhydre sous forme de poudre sont ajoutés. Le milieu est inerté sous azote. Pour démarrer la réaction d'éthérification (glycosylation) 0,2 g d'une solution aqueuse de H₃PO₂ à 50 % puis 0,3 g d'une solution aqueuse de H₂SO₄ à 98 % sont ajoutés et la température est augmentée et maintenue à 105°C. La réaction est poursuivie pendant 5h00.

### ETAPE 2 : Neutralisation du milieu réactionnel :

Le milieu est refroidi à 70°C à pression atmosphérique puis filtré sur filtre K1 (4 µm) afin d'éliminer le sucre résiduel. Le produit (160 g) est ensuite réintroduit en réacteur à 75°C puis neutralisé en introduisant sous agitation 1,77 g d'une solution aqueuse de Na₂CO₃ à 10 % pour atteindre une composition référencée (Composition 5).

### Analyses :

- La valeur du pH d'une dispersion à 5% massique dans l'eau de la (Composition 5) est de 6,9, et
- la mesure de la couleur de (Composition 5) est de 1,0 VCS.

### 2. Exemples comparatifs

### Exemple 2.1 : exemple comparatif (Coupe d'alcools 16/18 et NaOH comme agent pré-neutralisant et NaOH comme agent neutralisant).

### ETAPE 1 : Réaction de Glycosylation :

529,3 g d'alcool cétéarylique (C-16/18) sont chargés dans un réacteur équipé d'une agitation mécanique et d'un montage de distillation sous vide. L'alcool est fondu à 85°C et mis sous agitation et sous barbotage d'azote. Le milieu est mis sous vide à des pressions inférieures à 50 millibars. 69,5 g de glucose anhydre sous forme de poudre sont ajoutés. Le milieu est inerté sous azote. Pour démarrer la réaction d'éthérification (glycosylation) 0,5 g d'une solution aqueuse de H₃PO₂ à 50 % puis 0,6 g d'une solution aqueuse de H₂SO₄ à 98 % sont ajoutés et la température est augmentée et maintenue à 105°C. La réaction est poursuivie pendant 5h45.

### ETAPE 2 : Neutralisation du milieu réactionnel :

Le milieu est refroidi à 80°C à pression atmosphérique puis filtré sur filtre cloche équipé d'une plaque (3-6 µm) afin d'éliminer le sucre résiduel. 500 g de produit sont ensuite réintroduits en réacteur à 85°C puis neutralisés en ajoutant sous agitation 0,81 g d'une solution aqueuse de NaOH à 40 % pour atteindre une composition référencée (Composition 1').

### Analyses :

- La valeur du pH d'une dispersion à 5% massique dans l'eau de la (Composition 1') est de 6,5, et
- la mesure de la couleur de la (Composition 1') est de 2,8 VCS.

**[Tableau1]**

| Référence | Composition 1' | Composition 1 | Composition 2 | Composition 3 | 5898 JG2 Composition 4 | 5918 JG |
|---|---|---|---|---|---|---|
| Chaîne alkyle APG | C-16/18 | | | C-14 | | C-12 |
| Agent basique Pré-neutralisant | NaOH | Na₂CO₃ | NaOH | NaOH | Na₂CO₃ | Na₂CO₃ |
| Agent basique Neutralisant | NaOH | Na₂CO₃ | Na₂CO₃ | Na₂CO₃ | Na₂CO₃ | Na₂CO₃ |
| pH dispersion 5% dans l'eau | 6,5 | 6,4 | 6,4 | 6,6 | 7,4 | 6,9 |
| Couleur (vcs) | 2,8 | 0,9 | 1,0 | 1,1 | 1,0 | 1,0 |

Ces essais mettent en évidence que :
- la neutralisation jusqu'à un pH de la dispersion à 5% dans l'eau compris entre 5,5 et 7,5 par le seul carbonate de sodium permet d'atteindre le niveau de coloration recherché (inférieur ou égal à 1,5 VCS pour (Composition 1)) alors que la neutralisation jusqu'à un pH de la dispersion à 5% dans l'eau compris entre 5,5 et 7,5 par la seule soude aboutit à une coloration supérieure à 1,5 VCS, en l'occurrence jusqu'à environ 2,8 VCS (Composition 1').
- lorsque l'étape de pré-neutralisation est réalisée avec une solution de NaOH ou Na₂CO₃ et que l'étape de neutralisation est réalisée avec une solution aqueuse de Na₂CO₃ les couleurs des produits obtenus sont très basses (inférieures ou égales à 1,5 vcs).

Il a donc été montré qu'il est possible de neutraliser les compositions obtenues par la mise en œuvre du procédé selon l'invention par une solution de Na₂CO₃, dans une gamme de pH comprise entre 5,5 à 7,5 pour une dispersion à 5% dans l'eau des dites compositions, après filtration des sucres résiduels sans augmenter la couleur des compositions à une valeur supérieure à 1,5 VCS.

## Revendications

1. Procédé de préparation d'une composition (C) de couleur inférieure ou égale à 1,5 vcs, comprenant pour 100% de sa masse :
(i) une quantité supérieure ou égale à 40% massique et inférieure ou égale à 95% massique d'un alcool de formule (I) :
R-OH (I), dans laquelle R représente un radical hydrocarboné, linéaire ou ramifié, saturé ou insaturé, pouvant comporter au moins un fonction hydroxy, et comportant de douze à vingt-deux atomes de carbone, ou d'un mélange d'alcools de formule (I) ;
(ii) une quantité supérieure ou égale à 5% massique et inférieure ou égale à 60% massique, d'une composition (C1) représentée par la formule (II) :
R-O-(G)x-H (II), dans laquelle le reste G représente le reste d'un sucre réducteur, R représente un radical tel que défini dans la formule (I) et x, qui indique le degré moyen de polymérisation du reste G représente un nombre décimal supérieur à 1,05 et inférieur ou égal à 2,5, ou d'un mélange de compositions (C1) de formule (II); étant entendu que la somme des proportions massiques des composés et des compositions (I) et (II) est égale à 100% massique,
ledit procédé comprenant successivement :
a) Une étape a) de glycosylation, consistant en une réaction entre au moins un alcool de formule (I) et au moins un sucre réducteur de formule (III) : H-O-(G)-H (III), en présence d'au moins un catalyseur acide (CA), à une température supérieure ou égale à 100°C et inférieure ou égale à 120°C, le au moins un catalyseur acide (CA) étant choisi parmi les éléments du groupe constitué par l'acide sulfurique, l'acide chlorhydrique, l'acide phosphorique, l'acide nitrique, l'acide hypophosphoreux, l'acide méthane-sulfonique, l'acide para-toluène sulfonique, l'acide trifluorométhane sulfonique et les résines échangeuses d'ions acides,
b) Une étape b) d'élimination du sucre réducteur de formule (III), qui n'a pas réagi à l'étape a), du milieu réactionnel,
c) Une étape c) de neutralisation du milieu réactionnel issu de l'étape b) avec une solution aqueuse comprenant un agent basique (Ab), l'agent basique (Ab) étant choisi parmi les éléments du groupe constitué par :
▪ les carbonates de formule (IVa) :
XnCO₃ (IVa),
dans laquelle X représente un atome de sodium ou de potassium et n est un nombre entier égal à 2, ou X représente un atome de calcium ou un atome de magnésium et n est un nombre entier égal à 1, ou
▪ les hydrogénocarbonates de formule (IVb) :
Y(HCO₃)m (IVb),
dans laquelle Y représente un atome de sodium ou de potassium et m est un nombre entier égal à 1, ou Y représente un atome de calcium ou un atome de magnésium et m est un nombre entier égal à 2,
la neutralisation étant réalisée de manière à obtenir un milieu réactionnel dont la dispersion à 5% massique dudit milieu réactionnel dans l 'eau présente une valeur de pH comprise entre 5,5 et 7,5, et
d) Une étape d) de récupération d'au moins une composition (C) de couleur inférieure ou égale à 1,5 vcs.

2. Procédé selon la revendication 1, **caractérisé en ce que** ladite composition (C1) consistant en un mélange de composés représentés par les formules (II1), (II2), (II3), (II4) et (II5):
R-O-(G)1-H (II1),
R-O-(G)2-H (II2),
R-O-(G)3-H (II3),
R-O-(G)4-H (II4),
R-O-(G)5-H (II5),
dans les proportions molaires respectives a1, a2, a3, a4 et a5, telles que:
▪ la somme: a1+ a2 + a3 + a4 + a5 est égale à 1, et
▪ la somme a1 + 2a2 + 3a3 + 4a4 + 5a5 est égale à x.

3. Procédé selon l'une des revendications 1 ou 2, **caractérisé en ce que** la composition (C) comprend une quantité inférieure ou égale à 2% massique, de préférence inférieure ou égale à 1% massique du sucre réducteur de formule (III) :
H-O-(G)-H (III) ;
étant entendu que la somme des proportions massiques des composés et des compositions (I), (II) et (III) est égale à 100% massique.

4. Procédé selon l'une des revendications 1 à 3, **caractérisé en ce que** l'agent basique (Ab) présent dans la solution aqueuse est le carbonate de sodium de formule (IVa) dans laquelle X représente l'atome de sodium et n est égal à 2.

5. Procédé selon l'une des revendications 1 à 4, **caractérisé en ce qu'**à l'étape c) la solution aqueuse de l'agent basique (Ab) comprend entre 10 % et 25% massique dudit agent basique (Ab).

6. Procédé selon l'une des revendications 1 à 5, **caractérisé en ce que** l'étape c) est réalisée en deux temps : un premier temps durant lequel la neutralisation est réalisée de manière à obtenir un milieu réactionnel dont une dispersion à 5% massique dans l'eau présente une valeur du pH comprise entre 3,5 et 5,5 avec une solution d'hydroxyde de sodium (NaOH) ou d'hydroxyde de potassium (KOH), puis un deuxième temps durant lequel la neutralisation est réalisée de manière à obtenir un milieu réactionnel dont une dispersion à 5% massique dans l' eau présente une valeur du pH compris entre 5,5 et 7,5 avec une solution aqueuse d'agent basique (Ab) .

7. Procédé selon l'une des revendications 1 à 6, **caractérisé en ce que** le sucre réducteur de formule (III) choisi pour la glycolysation de l'étape a) est choisi parmi les éléments du groupe constitué par le glucose, le xylose, l'arabinose et le rhamnose.

8. Procédé selon l'une des revendications 1 à 7, **caractérisé en ce que** l'étape b) d'élimination du sucre réducteur de formule (III) est réalisée par filtration, centrifugation ou décantation.

9. Procédé selon l'une des revendications 1 à 8, **caractérisé en ce que** l'étape a) comprend les sous-étapes successives suivantes :
i) Introduction d'un alcool de formule (I) dans un réacteur (Ré) équipé d'une agitation mécanique et d'un dispositif de mise sous vide ;
ii) Chauffage de l'alcool de formule (I) à une température comprise entre 80°C et 90°C. sous agitation mécanique ;
iii) Chargement du sucre réducteur de formule (III) dans le réacteur (Ré) ;
iv) Introduction d'au moins un catalyseur (CA) dans le réacteur (Ré),
v) Chauffage sous vide du milieu réactionnel présent dans le réacteur (Ré) à une température comprise entre 100°C et 110°C pendant la durée de la réaction, et
vi) Refroidissement du milieu issu de la sous-étape v) à une température comprise entre 70°C et 80°C.

10. Procédé selon la revendication 9, **caractérisé en ce que** dans la formule (I) et/ou dans la formule (II) le radical R est choisi parmi les radicaux suivants : lauryle (ou n-dodécyle) myristyle (ou n-tétradécyle ), n-pentadécyle, cétyle (ou n-hexadécyle), n-heptadécyle, stéaryle (ou n-octadécyle), palmitoléyle (ou 9-hexadécényle), oléyle (ou 9-octadécényle), linoléyle (9,12-octadecadiényle), linolényle (ou 6,9,12-octadécatriényle) nonadécyle, arachidyle (ou n-eicosyle), béhényle (ou n-docosyle), érucyle (13-docosényle), ou 12-hydroxystéaryle.

11. Procédé selon la revendication 9, **caractérisé en ce que** dans la formule (I) et/ou dans la formule (II) le radical R est choisi parmi les radicaux suivants : 2-hexyl octyle, 2-hexyl décyle, 2-hexyl dodécyle, 2-octyl décyle, 2-octyl dodécyle, 2-décyl tétradécyle, isostéaryle (ou 16-méthyl heptadécyle) ou isomyristyle (ou 13-méthyl tridécyle).

12. Procédé selon l'une des revendications 9 à 11, **caractérisé en ce que** pendant les sous- étapes ii) à iv) le réacteur (Ré) est inerté sous azote.

13. Procédé selon l'une des revendications 9 à 12, **caractérisé en ce qu'**il comprend entre les sous-étapes ii) et iii) une étape de mise sous vide, de préférence à une pression inférieure ou égale à 50 millibars.

14. Procédé selon l'une des revendications 9 à 13, **caractérisé en ce que** la sous-étape vi) est réalisée à pression atmosphérique.

## Patentansprüche

1. Verfahren zur Herstellung einer Zusammensetzung (C) mit einer Farbe von höchstens 1,5 VCS, umfassend, bezogen auf 100 Gew.-% ihrer Masse:
(i) eine Menge von mindestens 40 Gew.-% und höchstens 95 Gew.-% eines Alkohols der Formel (I):
R-OH (I), wobei R einen linearen oder verzweigten, gesättigten oder ungesättigten Kohlenwasserstoffrest darstellt, der mindestens eine Hydroxyfunktion umfassen kann und zwölf bis zweiundzwanzig Kohlenstoffatome aufweist, oder eine Mischung von Alkoholen der Formel (I);
(ii) eine Menge von mindestens 5 Gew.-% und höchstens 60 Gew.-% einer Zusammensetzung (C1), dargestellt durch die Formel (II):
R-O-(G)x-H (II), wobei der Rest G den Rest eines reduzierenden Zuckers darstellt, R einen Rest wie in Formel (I) definiert darstellt und x, das den mittleren Polymerisationsgrad des Restes G angibt, eine Dezimalzahl größer als 1,05 und kleiner oder gleich 2,5 darstellt, oder eine Mischung von Zusammensetzungen (C1) der Formel (II); wobei davon ausgegangen wird, dass die Summe der Massenanteile der Verbindungen und der Zusammensetzungen (I) und (II) gleich 100 Gew.-% ist,
wobei das Verfahren nacheinander umfasst:
a) Einen Glykosylierungsschritt a), bestehend aus einer Reaktion zwischen mindestens einem Alkohol der Formel (I) und mindestens einem reduzierenden Zucker der Formel (III): H-O-(G)-H (III), in Gegenwart von mindestens einem sauren Katalysator (CA), bei einer Temperatur von mindestens 100°C und höchstens 120°C, wobei der mindestens eine saure Katalysator (CA) ausgewählt ist aus der Gruppe bestehend aus Schwefelsäure, Salzsäure, Phosphorsäure, Salpetersäure, hypophosphoriger Säure, Methansulfonsäure, para-Toluolsulfonsäure, Trifluormethansulfonsäure und sauren Ionenaustauscherharzen,
b) Einen Schritt b) der Eliminierung des reduzierenden Zuckers der Formel (III), der in Schritt a) nicht reagiert hat, aus dem Reaktionsmedium,
c) Einen Schritt c) der Neutralisation des aus Schritt b) resultierenden Reaktionsmediums mit einer wässrigen Lösung, umfassend ein basisches Mittel (Ab), wobei das basische Mittel (Ab) ausgewählt ist aus den Elementen der Gruppe bestehend aus:
* Carbonaten der Formel (IVa): XnCO₃ (IVa), wobei X ein Natriumatom oder ein Kaliumatom darstellt und n eine ganze Zahl gleich 2 ist, oder X ein Calciumatom oder ein Magnesiumatom darstellt und n eine ganze Zahl gleich 1 ist, oder
* Hydrogencarbonaten der Formel (IVb): Y(HCO₃)m (IVb), wobei Y ein Natriumatom oder ein Kaliumatom darstellt und m eine ganze Zahl gleich 1 ist, oder Y ein Calciumatom oder ein Magnesiumatom darstellt und m eine ganze Zahl gleich 2 ist,
wobei die Neutralisation so durchgeführt wird, dass ein Reaktionsmedium erhalten wird, dessen 5 Gew.-%ige Dispersion in Wasser einen pH-Wert zwischen 5,5 und 7,5 aufweist, und
d) Einen Schritt d) zur Gewinnung mindestens einer Zusammensetzung (C) mit einer Farbe von höchstens 1,5 VCS.

2. Das Verfahren nach Anspruch 1, **dadurch gekennzeichnet, dass** die besagte Zusammensetzung (C1) aus einer Mischung von Verbindungen besteht, die durch die Formeln (II1), (II2), (II3), (II4) und (II5) dargestellt werden:
R-O-(G)1-H (II1),
R-O-(G)2-H (II2),
R-O-(G)3-H (II3),
R-O-(G)4-H (II4),
R-O-(G)5-H (II5),
in den jeweiligen molaren Anteilen a1, a2, a3, a4 und a5, so dass:
* die Summe: a1+ a2 + a3 + a4 + a5 gleich 1 ist, und
* die Summe a1 + 2a2 + 3a3 + 4a4 + 5a5 gleich x ist.

3. Das Verfahren nach einem der Ansprüche 1 oder 2, **dadurch gekennzeichnet, dass** die Zusammensetzung (C) eine Menge von höchstens 2 Gew.-%, vorzugsweise höchstens 1 Gew.-% des reduzierenden Zuckers der Formel (III) umfasst:
H-O-(G)-H (III);
wobei davon ausgegangen wird, dass die Summe der Massenanteile der Verbindungen und der Zusammensetzungen (1), (II) und (III) gleich 100 Gew.-% ist.

4. Das Verfahren nach einem der Ansprüche 1 bis 3, **dadurch gekennzeichnet, dass** das in der wässrigen Lösung vorhandene basische Mittel (Ab) Natriumcarbonat der Formel (IVa) ist, wobei X das Natriumatom darstellt und n gleich 2 ist.

5. Das Verfahren nach einem der Ansprüche 1 bis 4, **dadurch gekennzeichnet, dass** in Schritt c) die wässrige Lösung des basischen Mittels (Ab) zwischen 10 Gew.-% und 25 Gew.-% des besagten basischen Mittels (Ab) umfasst.

6. Das Verfahren nach einem der Ansprüche 1 bis 5, **dadurch gekennzeichnet, dass** Schritt c) in zwei Schritten durchgeführt wird: ein erster Schritt, in dem die Neutralisation so durchgeführt wird, dass ein Reaktionsmedium erhalten wird, dessen 5 Gew.-%ige Dispersion in Wasser einen pH-Wert zwischen 3,5 und 5,5 mit einer Lösung von Natriumhydroxid (NaOH) oder Kaliumhydroxid (KOH) aufweist, gefolgt von einem zweiten Schritt, in dem die Neutralisation so durchgeführt wird, dass ein Reaktionsmedium erhalten wird, dessen 5 Gew.-%ige Dispersion in Wasser einen pH-Wert zwischen 5,5 und 7,5 mit einer wässrigen Lösung eines basischen Mittels (Ab) aufweist.

7. Das Verfahren nach einem der Ansprüche 1 bis 6, **dadurch gekennzeichnet, dass** der für die Glykosylierung von Schritt a) ausgewählte reduzierende Zucker der Formel (III) ausgewählt ist aus der Gruppe bestehend aus Glucose, Xylose, Arabinose und Rhamnose.

8. Das Verfahren nach einem der Ansprüche 1 bis 7, **dadurch gekennzeichnet, dass** Schritt b) der Eliminierung des reduzierenden Zuckers der Formel (III) durch Filtration, Zentrifugation oder Dekantierung durchgeführt wird.

9. Das Verfahren nach einem der Ansprüche 1 bis 8, **dadurch gekennzeichnet, dass** Schritt a) die folgenden aufeinanderfolgenden Teilschritte umfasst:
i) Einführung eines Alkohols der Formel (I) in einen Reaktor (Ré), der mit einem mechanischen Rührer und einer Vakuumeinrichtung ausgestattet ist;
ii) Erwärmen des Alkohols der Formel (I) auf eine Temperatur zwischen 80°C und 90°C unter mechanischem Rühren;
iii) Beschickung des reduzierenden Zuckers der Formel (III) in den Reaktor (Ré);
iv) Einführung mindestens eines Katalysators (CA) in den Reaktor (Ré),
v) Erwärmen des im Reaktor (Ré) vorhandenen Reaktionsmediums unter Vakuum auf eine Temperatur zwischen 100°C und 110°C während der Dauer der Reaktion, und
vi) Abkühlen des aus Teilschritt v) stammenden Mediums auf eine Temperatur zwischen 70°C und 80°C.

10. Das Verfahren nach Anspruch 9, **dadurch gekennzeichnet, dass** in der Formel (I) und/oder in der Formel (II) der Rest R ausgewählt ist aus den folgenden Resten: Lauryl (oder n-Dodecyl) Myristyl (oder n-Tetradecyl), n-Pentadecyl, Cetyl (oder n-Hexadecyl), n-Heptadecyl, Stearyl (oder n-Octadecyl), Palmitoleyl (oder 9-Hexadecenyl), Oleyl (oder 9-Octadecenyl), Linoleyl (9,12-Octadecadienyl), Linolenyl (oder 6,9,12-Octadecatrienyl) Nonadecyl, Arachidyl (oder n-Eicosyl), Behenyl (oder n-Docosyl), Erucyl (13-Docosenyl) oder 12-Hydroxystearyl.

11. Das Verfahren nach Anspruch 9, **dadurch gekennzeichnet, dass** in der Formel (I) und/oder in der Formel (II) der Rest R ausgewählt ist aus den folgenden Resten: 2-Hexyloctyl, 2-Hexyldecyl, 2-Hexyldodecyl, 2-Octyldecyl, 2-Octyldodecyl, 2-Decyltetradecyl, Isostearyl (oder 16-Methylheptadecyl) oder Isomyristyl (oder 13-Methyltridecyl).

12. Das Verfahren nach einem der Ansprüche 9 bis 11, **dadurch gekennzeichnet, dass** der Reaktor (Ré) während der Teilschritte ii) bis iv) unter Stickstoff inertisiert wird.

13. Das Verfahren nach einem der Ansprüche 9 bis 12, **dadurch gekennzeichnet, dass** es zwischen den Teilschritten ii) und iii) einen Schritt des Anlegens eines Vakuums umfasst, vorzugsweise bei einem Druck von höchstens 50 Millibar.

14. Das Verfahren nach einem der Ansprüche 9 bis 13, **dadurch gekennzeichnet, dass** der Teilschritt vi) bei atmosphärischem Druck durchgeführt wird.

## Claims

1. A method for preparing a composition (C) having a colour index lower than or equal to 1.5 VCS, comprising, for 100% of its mass:
(i) an amount greater than or equal to 40% by mass and less than or equal to 95% by mass of an alcohol of formula (I):
R-OH (I), in which R represents a hydrocarbon radical, linear or branched, saturated or unsaturated, potentially comprising at least one hydroxy function, and comprising from twelve to twenty-two carbon atoms, or a mixture of alcohols of formula (I);
(ii) an amount greater than or equal to 5% by mass and less than or equal to 60% by mass of a composition (C1) represented by formula (II):
R-O-(G)x-H (II), in which the residue G represents the residue of a reducing sugar, R represents a radical as defined in formula (I) and x, which indicates the average degree of polymerization of the residue G, represents a decimal number greater than 1.05 and less than or equal to 2.5, or a mixture of compositions (C1) of formula (II); it being understood that the sum of the mass proportions of compounds and compositions (I) and (II) is equal to 100% by mass,
said method successively comprising:
a) A glycosylation step a), consisting of a reaction between at least one alcohol of formula (I) and at least one reducing sugar of formula (III): H-O-(G)-H (III), in the presence of at least one acid catalyst (CA), at a temperature greater than or equal to 100°C and less than or equal to 120°C, the at least one acid catalyst (CA) being chosen from the elements of the group consisting of sulfuric acid, hydrochloric acid, phosphoric acid, nitric acid, hypophosphorous acid, methane sulfonic acid, para-toluene sulfonic acid, trifluoromethane sulfonic acid and acid ion exchange resins,
b) A step b) of eliminating the reducing sugar of formula (III), which has not reacted in step a), from the reaction medium,
c) A step c) of neutralization of the reaction medium resulting from step b) with an aqueous solution comprising a basic agent (Ab), the basic agent (Ab) being chosen from the elements of the group consisting of:
* carbonates of formula (IVa): XnCO₃ (IVa), in which X represents a sodium atom or a potassium atom and n is an integer equal to 2, or X represents a calcium atom or a magnesium atom and n is an integer equal to 1, or
* hydrogen carbonates of formula (IVb): Y(HCO₃)m (IVb), in which Y represents a sodium atom or a potassium atom and m is an integer equal to 1, or Y represents a calcium atom or a magnesium atom and m is an integer equal to 2,
the neutralization being carried out so as to obtain a reaction medium whose 5% by mass dispersion in water has a pH value between 5.5 and 7.5, and
d) A step d) of recovering at least one composition (C) having a colour index lower than or equal to 1.5 VCS.

2. The method according to claim 1, **characterized in that** said composition (C1) consists of a mixture of compounds represented by formulas (II1), (II2), (II3), (II4) and (II5):
R-O-(G)1-H (II1),
R-O-(G)2-H (II2),
R-O-(G)3-H (II3),
R-O-(G)4-H (II4),
R-O-(G)5-H (II5),
in the respective molar proportions a1, a2, a3, a4 and a5, such that:
* the sum: a1+ a2 + a3 + a4 + a5 is equal to 1, and
* the sum a1 + 2a2 + 3a3 + 4a4 + 5a5 is equal to x.

3. The method according to one of claims 1 or 2, **characterized in that** the composition (C) comprises an amount less than or equal to 2% by mass, preferably less than or equal to 1% by mass of the reducing sugar of formula (III):
H-O-(G)-H (III);
it being understood that the sum of the mass proportions of compounds and compositions (I), (II) and (III) is equal to 100% by mass.

4. The method according to one of claims 1 to 3, **characterized in that** the basic agent (Ab) present in the aqueous solution is sodium carbonate of formula (IVa) in which X represents the sodium atom and n is equal to 2.

5. The method according to one of claims 1 to 4, **characterized in that** in step c) the aqueous solution of the basic agent (Ab) comprises between 10% and 25% by mass of said basic agent (Ab).

6. The method according to one of claims 1 to 5, **characterized in that** step c) is carried out in two steps: a first step during which neutralization is carried out so as to obtain a reaction medium whose 5% by mass dispersion in water has a pH value between 3.5 and 5.5 with a solution of sodium hydroxide (NaOH) or potassium hydroxide (KOH), then a second step during which neutralization is carried out so as to obtain a reaction medium whose 5% by mass dispersion in water has a pH value between 5.5 and 7.5 with an aqueous solution of basic agent (Ab).

7. The method according to one of claims 1 to 6, **characterized in that** the reducing sugar of formula (III) chosen for the glycosylation of step a) is chosen from the elements of the group consisting of glucose, xylose, arabinose and rhamnose.

8. The method according to one of claims 1 to 7, **characterized in that** step b) of eliminating the reducing sugar of formula (III) is carried out by filtration, centrifugation or decantation.

9. The method according to one of claims 1 to 8, **characterized in that** step a) comprises the following successive sub-steps:
i) Introduction of an alcohol of formula (I) into a reactor (Ré) equipped with mechanical stirring and a vacuum device;
ii) Heating of the alcohol of formula (I) to a temperature comprised between 80°C and 90°C. under mechanical stirring;
iii) Charging of the reducing sugar of formula (III) into the reactor (Ré);
iv) Introduction of at least one catalyst (CA) into the reactor (Ré),
v) Heating under vacuum of the reaction medium present in the reactor (Ré) to a temperature comprised between 100°C and 110°C during the duration of the reaction, and
vi) Cooling of the medium resulting from sub-step v) to a temperature comprised between 70°C and 80°C.

10. The method according to claim 9, **characterized in that** in formula (I) and/or in formula (II) the radical R is chosen from the following radicals: lauryl (or n-dodecyl) myristyl (or n-tetradecyl), n-pentadecyl, cetyl (or n-hexadecyl), n-heptadecyl, stearyl (or n-octadecyl), palmitoleyl (or 9-hexadecenyl), oleyl (or 9-octadecenyl), linoleyl (9,12-octadecadienyl), linolenyl (or 6,9,12-octadecatrienyl) nonadecyl, arachidyl (or n-eicosyl), behenyl (or n-docosyl), erucyl (13-docosenyl), or 12-hydroxystearyl.

11. The method according to claim 9, **characterized in that** in formula (I) and/or in formula (II) the radical R is chosen from the following radicals: 2-hexyl octyl, 2-hexyl decyl, 2-hexyl dodecyl, 2-octyl decyl, 2-octyl dodecyl, 2-decyl tetradecyl, isostearyl (or 16-methyl heptadecyl) or isomyristyl (or 13-methyl tridecyl).

12. The method according to one of claims 9 to 11, **characterized in that** during sub-steps ii) to iv) the reactor (Ré) is inerted under nitrogen.

13. The method according to one of claims 9 to 12, **characterized in that** it comprises between sub-steps ii) and iii) a vacuum application step, preferably at a pressure less than or equal to 50 millibars.

14. The method according to one of claims 9 to 13, **characterized in that** sub-step vi) is carried out at atmospheric pressure.
